# EUROPEAN PATENT APPLICATION

(11) **EP 2 319 510 A1**
(43) Date of publication of application: **11.05.2011**
(21) Application number: 10251713.3
(22) Date of filing: 01.10.2010
(51) Int. Cl.: A61K 31/327, A61K 9/08, A61K 47/34, A61K 47/44, A61K 9/00, A61P 17/10

(54) **Benzoyl peroxide composition for treating skin**

(30) Priority: 02.10.2009 US 572435
(71) Applicant: Johnson and Johnson Consumer Companies, Inc., Skillman, NJ 08558 (US)
(72) Inventor: Wu, Jeffrey M., Princeton, NJ 08540 (US); Sasik, Camille, Belle Mead, NJ 08502 (US)
(74) Representative: Kirsch, Susan Edith

(57) **Abstract**

This invention relates to a novel composition useful for the treatment of skin, and more particularly, to a composition containing benzoyl peroxide for treating acne while providing enhanced delivery and low level of irritation to skin.
The composition comprises a mixture of a co-solvent (preferably volatile polysiloxanes) and an ester (like for instance waxes, shea butter, cetyl stearate, sunflowerseedcetyl lactate).

## Description

### FIELD OF THE INVENTION

This invention relates to a novel composition useful for the treatment of skin, and more particularly, to a composition containing benzoyl peroxide for treating acne while providing a low level of irritation to skin.

### BACKGROUND OF THE INVENTION

Acne and sebhorrea are conditions of the human skin characterized by an excessive flow of sebum or skin oil, from the sebaceous glands which are located in the pilosebaceous apparatus. The channel through which sebum reaches the skin surface is the duct of the hair follicle. The presence of excess amounts of sebum in the duct and on the skin acts to block or stagnate the continuous flow of sebum from the follicle duct, thus producing a thickening of sebum which becomes a solid plug known as a comedone. When this occurs, hyperkeratinization of the follicular opening is stimulated, thus completely closing the duct. The usual result is a papule, pustule or a cyst, which are often contaminated with bacteria that cause secondary infections. These occurences characterize the disease today known as acne, and in lesser severity, sebhorrea.

Skin care acne treatments should be both effective and well tolerated. Because of the prevalence and chronicity of acne, as well as its psychological impact, the availability of an effective and well-tolerated treatment is critical. Burning, dryness and peeling caused by acne treatments such as benzoyl peroxide may be as bothersome to some patients as the acne blemishes themselves, and may result in non-compliance. This decreased compliance can lead to decreased efficacy. This has limited most of the current benzoyl peroxide based products as a spot treatment to minimize the irritation risks.

U.S. Patent Appln. Publication No. 2006/0008538A1, filed on June 20, 2005, which is incorporated herein by reference, discloses compositions comprising an anti-acne agent, an anti-microbial agent and a lactate. Applicants have now discovered means for mitigating irritation of lipophilic anti-acne agents, such as benzoyl peroxide, improving follicular delivery of such lipophilic compounds and providing effective acne treatment.

### SUMMARY OF THE INVENTION

The present invention features a composition for treating acne comprising an anti-acne agent, a mixture of a co-solvent and an ester, and a topical carrier, wherein said ester has a melting point of at least about 30° C and said mixture is substantially immiscible with said topical carrier.

The present invention also features a method of treating a follicular disease comprising topically applying to an area of skin in need of such treatment a composition comprising an anti-acne agent, a mixture of a co-solvent and an ester, and a topical carrier, wherein said ester has a melting point of at least about 30° C and said mixture is substantially immiscible with said topical carrier.

Other features and advantages of the present invention will be apparent from the detailed description of the invention and from the claims.

### BRIEF DESCRIPTION OF THE DRAWINGS

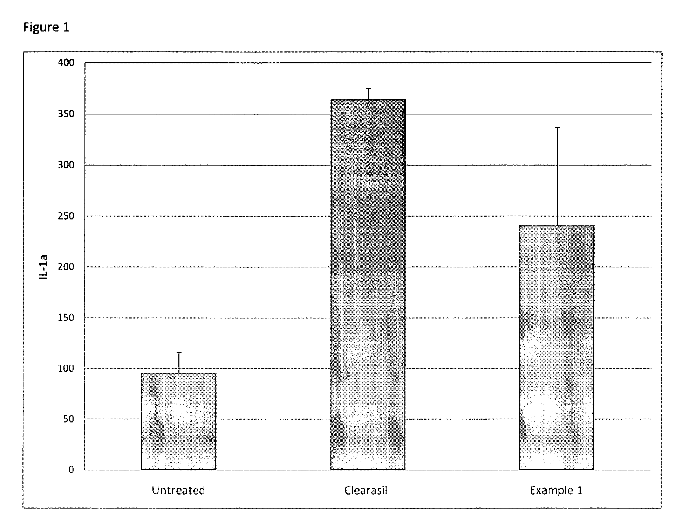

Figure 1 shows the IL-1α response in skin equivalent samples described in Example 4.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Whenever used, any percentage is weight by weight (w/w) unless otherwise indicated.

As used herein, "follicular disease" means a condition or disease affecting follicles, such as acne, rosacea, folliculitis, follicular keratosis and telogenization, follicular hyperkeratosis or phrynoderma, ichthyosis follicularis, alopecia and follicular dysplasia, hirutism/hypertrichosis, follicular infundibulum, fungal pimples, dandruff and seborrhea.

As used herein, "topically applying" means directly laying on or spreading on outer skin, e.g., by use of the hands or an applicator such as a wipe, puff, roller, or spray.

As used herein, "cosmetically-acceptable" means that the compound(s) or composition(s) which the term describes are suitable for use in contact with tissues (e.g., the skin) without undue toxicity, incompatibility, instability, irritation, allergic response, and the like. This term is not intended to limit the compound/composition to which it describes for use solely as a cosmetic (e.g., the ingredient/product may be used as a pharmaceutical).

As used herein, "safe and effective amount" means an amount of compound(s) or composition(s) sufficient to treat acne, but low enough to avoid serious side effects.

What is meant by "promoting" is promoting, advertising, or marketing. Examples of promoting include, but are not limited to, written, visual, or verbal statements made on the product containing the composition or in stores, magazines, newspaper, radio, television, internet, and the like.

The compositions of the present invention are useful for treating follicular diseases, such as acne, rosacea, hyperlipidemia, seborrhea, dandruff, sebacious hyperplasia, follicular rash, demodex folliculorum follicular infections such as folliculitis, staphylcoccal impetigoacne necrotica, and psudofolliculitis barbe, follicular ketarosis and telogenization, keratosis pilaris, follicular hyperkeratosis or phrynoderma, ichthyosis follicularis, alopecia, follicular dysplasia, hirsutism, oily skin, hypertrichosis, follicular infundibulum, and fungal pimples.

As used herein, the term "treating" or "treatment" means the treatment (e.g., alleviation or elimination of symptoms and/or cure) and/or prevention or inhibition of the condition (e.g., a skin condition).

The compositions of the present invention are also useful for evening skin tone (such as lightening dark areas of skin) in need of such treatment, smoothing the skin (such as reducing texture on the skin), reducing the production of sebum, and reducing the appearance of oil, shine, and/or pores on skin in need of such treatment. Examples of such skin in need of such treatment include, but are not limited to, skin having excessive pigmentation (such as freckles, post-inflammatory hyperpigmentation (PIH), or pigmented scars), rough skin, oily skin, or skin having large, visible pores.

In one embodiment, the composition is for the treatment of acne, including but not limited to the treatment or prevention of acne blemishes, acne pimples, pre-emergent pimples, blackheads, and/or whiteheads. What is meant by a "pre-emergent pimple" is an inflamed follicle that are not visually apparent on the surface of the skin with the naked eye (e.g., as a lesion).

In one embodiment, the present invention relates to compositions including an anti-acne agent. What is meant by an anti-acne agent is a compound that has been approved by the U.S. Food and Drug Administration for the topical treatment of acne. Examples of anti-acne agents include, but are not limited to, salicylic acid, benzoyl peroxide, sulphur, retinoic acid, candida bombicola/glucose/methyl rapeseedate ferment, peat water, resorcinol, silt, peat, permethin, azelaic acid, clindamycin, adapalene, erythromycin, sodium sulfacetamide, minocycline, tetracycline, oxycycline, sodium sulfacetamide, dapsone, retinoid such as isotretinoin, tretinoin, ethinyl estradiol, norgestimate, nicotinamide, and their derivatives, and combinations thereof. In one embodiment, the amount of anti-acne agent in the composition is from about 0.001% to about 30%, such as from about 0.01% to about 15%, for example from about 0.1% to about 5%, or from about 0.5% to about 2% by weight, based on the total weight of the composition.

The composition also comprises a mixture of a co-solvent and an ester.

In one embodiment, the co-solvent has a polarity parameter from about 5 to about 10.

In another embodiment, the co-solvent can be selected from volatile silicone and low flashing point emollients. Other non-limiting examples of co-solvents include, but are not limited to aliphatic hydrocarbons such as isodecan, isohexyl decan, isoparaffin, alkanes, esters formed from glycerol and fatty acids, and combinations thereof. The preferred range of co-solvent is from about 0.1 % to about 30%, or from about 0.5% to about 10% by weight, based on the total weight of the composition. Preferably an amount that substantially or completely covers or encapsulates the anti-acne agent is used.

Esters of the present invention have a melting point of at least about 30°C. Examples of esters include, but are not limited to C 16 or longer linear carbon chain alkyl lactates, hydrocarbon waxes, rice bran wax, beeswax, copernicia cerifera (carnauba) wax, sunflowseedcetyl lactate, myristate lactate, wax, euphobia wax, shea butter, cetyl stearate, cetyl alcohol, ethylhexylglycerin, petrolatum, cholesterol, their derivatives, and combinations thereof. The preferred range of esters used is from about 0.1% to about 15% or from about 0.5% to about 5% by weight, based on the total weight of the composition.

Advantageously, according to the invention, the anti-acne agent is delivered to a greater number of follicles, and delivered more deeply into the follicles. In particular, the number of follicles to which the anti-acne agent is delivered is increased by at least about 50%, for example at least 65%, by topical application of an anti-acne agent in combination with a mixture of co-solvent and ester according to the invention, compared topical application of the same anti-acne agent but not combined with a mixture of co-solvent and ester to the same size area of the skin.

In one embodiment, the anti-acne agent is benzoyl peroxide and the co-solvent is a volatile silicone. The benzoyl peroxide particles are pre-coated with a combination of the volatile silicone and an emollient having a polarity parameter of 6 to 10. The volatile silicone has a flashing point of lower than 150° C, preferably lower than 120° C. The volatile silicone may be, for example, a volatile cyclic polysiloxane such as a cyclic silicone containing from 4 to 6 siloxane groups or a linear or branched polysiloxane containing methyl groups, hexamethyldisiloxane (DC 200 Fluid 0.65 cst Dow Corning), octamethyltrisiloxane (DC 200 Fluid 1 cst Dow Corning); 1,1,1,3,5,5,5-heptamethyl-3-octyltrisiloxane, 1,1,1,3,5,5,5-heptamethyl-3-hexyltrisiloxane (DC2-1731, Dow Corning); decamethyltetrasiloxane (DC 200 Fluid 1.5 cst Dow Corning); a pentasiloxane, dodecamethylpentasiloxane ( DC 200 Fluid 2 cst Dow Corning); 1,1,1,5,5,5-hexamethyl-3-phenyl-3(trimethylsilyl)oxy]trisiloxane (Dow 556 Fluid); and those described for example in US Patent No. 5084577, and combinations thereof.

In one embodiment, the composition of the present invention further comprises a phospholipid. Examples of phospolipids include, but are not limited to synthetic phospholipids and natural phospholipids such as phospholipids composed of diester and triester phosphatides such as cocamidopropyl PG-dimonium chloride (Colalipid® C, Colonial Chemical, Inc., South Pittsburgh, Tenn., USA), stearamidopropyl PG-dimonium chloride (Colalipid® ST), sunfloweramidopropyl phosphate PG-dimonium chloride (Colalipid® SUN), sodium olivamidopropyl PG-dimonium chloride phosphate (Colalipid® OL), sodiumgrapeseedamindopropyl PG-dimonium chloride phosphate (Colalipid® GS), linoleamidopropyl PG-dimonium chloride phophate (Colalipid® SAFEL), PEG-8 dimethicone sunfloweramidopropyl PG-dimonium complex (Colalipid® SIL), ricinoleamidopropyl PG-dimonium chloride phosphate (Colalipid® RC), sodium coco PG-dimonium chloride phosphate (Arlasilk® phospholipids CDM, Uniqema, ICI Group of Companies, Wilton, UK), cocamidopropyl PG-dimonium chloride (Arlasilk® phospholipids PTC), stearamidipropyl PG-dimonium chloride phosphate (Arlasilk® phospholipids SV), linoleamidopropyl PG-dimonium chloride phosphate (Arlasilk® phospholipids EFA), linoleamidopropyl PG-dimonium chloride phosphate dimethicone(Arlasilk® phospholipids PLN), myristamidopropyl PG-dimonium chloride phosphate (Arlasilk® phospholipids PTM), and sodium borageamidopropyl PG-dimonium chloride phosphate (Arlasilk® phospholipids GLA), and combinations thereof.

In one embodiment, the composition of the present invention comprises a high melting point lactate. Examples of such lactates include, but are not limited to, long carbon chain lactates such as C16-C35 lactates, such as cetyl lactate. The amount of such lactates in the composition of the present invention may vary from about 0.1% to about 50%, for example from about 0.5% to about 20%, or from about 1% to about 10% by weight, based on the total weight of the composition.

In one embodiment, the composition includes a sebum miscible agent. What is meant by a sebum miscible agent is an agent that is miscible with sebum as determined by the following assay. Artificial sebum is prepared as set forth on page 79 (Table 5.4) of a book chapter entitled "The Influence of Skin Surface Lipids on Topical Formulations" by Obsorne and Hatzenbuhler (in "Topical Drug Delivery Formulations", edited by D. Osborne and A. A mann, Marcel Dekker, Inc., New York, 1990, pages 69-85). At room temperature this sebum is a white waxy substance. 50 .mu.l of the sebum is deposited into a 200 .mu.l clear vial using a precision micropipette. 100 .mu.l of the test agent is then added to the vial. The vial is warmed at 32.degree. C. and visually inspected at the baseline and at eight hours. If the agent is miscible with the sebum, the sebum will become transparent.

The following are non-limiting examples of sebum miscible agents: aromatic alcohols such as phenyl alcohols with chemical structures of C₆H₅-R(OH) where R is an aliphatic radical, such as benzyl alcohol and phenethyl alcohol; aromatic glycol ethers such as ethylene glycol phenyl ether; propylene or butylene oxide-based glycol ethers such as propylene glycol methyl ether and those disclosed in U.S. Pat. No. 5,133,967; fatty acids, polyunsaturated fatty acids such as linoleic acid, linolenic acid, stearidonic acid, plant, fruit, or marine derived extracts rich in essential fatty acid or polyunsaturated fatty acids such as but not limited to vaccinium myrtillus (bilberry) seed oil, vaccinium macrocarpon (cranberry) seed oil, vaccinium vitisidaea (lingonberry) seed oil, rubus idaeus (raspberry) seed oil, rubus chamaemorus (cloudberry) seed oil, ribes nigrum (black currant) seed oil, hippophae rhamnoides (sea buckthorn) seed oil, echium plantagineum (echium) seed oil, hordeum vulgare (barley) seed oil, betula alba bud extract, saw palmetto extract, borage oil, evening primrose oil, witch hazel extract and soy oil; cetyl ocenate; isostearyl benzoate; pentaerythiol teraoctenate; isostearyl benzoate; methyl gluceth; tocopherol acetate; benzalkonium chloride; and benzethonium chloride, and combinations thereof.

In one embodiment, the compositions of the present invention include an antimicrobial agent. What is meant by an antimicrobial agent is a compound that kills microorganisms or prevents or inhibits their growth or reproduction. Examples of antimicrobial agents include, but are not limited to: ethanol, propanol, betains, benzalkonium chloride, benzethonium chloride, lauric arginayte, sugarquat, methyl benzethonium chloride, cetypyridiunium chloride, 2,4,4',-trichloro-2-hydroxy diphenyl ether (Triclosan), parachlorometa xylenol (PCMX), lodopropynyl butylcarbamate, diazolidinyl urea, chlorhexidene digluconate, chlorhexidene acetate, chlorhexidene isethionate, chlorhexidene hydrochloride, hexetidine, Quaternium 15, triclocarbon, polyhexamethylene biguanide, cetylpyridium chloride, imidazolidinyl urea, diazolidinyl urea, 3-iodo-2-propynyl-N-butylcarbamate, 2-methyl-4-isothiazolin-3-one, dimethyl dimethyl hydantoin,(5-chloro-2-(2,4-dichlorophenoxy)phenol, monolaurin glyceryl laurate, camellia sinensis, candida bombicola/glucose/methyl rapeseedate ferment, hydrogen peroxide, phenol, poloxamer 188, PVP-iodine, thiourea, natural antimicrobial agents, such as cinnamon oil, cinnamaldehyde, lemongrass oil, clove oil, saw palmetto extract, thyme oil white, thyme oil red, thymol, tea tree oil, pinus pinaster bark extract, rosemary leaf extract, grape seed extract, and betel oil, silver containing compounds, such as silver nitrate, silver lactate, silver citrate, and silver zeolite, antimicrobial fatty acid ester of a polyhydric alcohol, a fatty ether of a polyhydric alcohol and alkoxylated derivatives thereof, and combinations thereof.

In one embodiment, the amount of antimicrobial agent in the composition is from about 0.001% to about 10%, such as from about 0.01% to about 5% such as from about 0.05% to about 2% by weight, based on the total weight of the composition.

In one embodiment the antimicrobial agent is an anti-fungal agent such as an azole. Examples include, but are not limited to, miconazole, ketoconazole, econazole, itraconazole, sertaconazole, fluconazole, voriconazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, their cosmetically acceptable salts, and combinations thereof.

In one embodiment the antimicrobial agent is an antibiotic or an antiseptic. Examples include, but are not limited to, mupirocin, neomycin sulfate bacitracin, polymyxin B, 1-ofloxacin, tetracyclines such as chlortetracycline hydrochloride, oxytetracycline-10 hydrochloride and tetrachcycline hydrochoride, clindamycin phosphate, gentamicin sulfate, metronidazole, hexylresorcinol, methylbenzethonium chloride, phenol, quaternary ammonium compounds, tea tree oil, and combinations thereof.

In one embodiment, the compositions of the present invention include an antipsoriatic agent. Examples of antipsoriatic agents include, but are not limited to, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone venerate, hydrocortisone butyrate, aclometasone dipropionte, flurandrenolide, mometasone furoate, and methylprednisolone acetate), methotrexate, cyclosporine, calcipotriene, anthraline, shale oil, elubiol, ketoconazole, coal tar, salicylic acid, zinc pyrithione, selenium sulfide, hydrocortisone, sulfur, , 2,2'-sulfanediylbis(4,6-dichlorophenol) (bithionol), 6-hydroxy-1,3-benzoxathiol-2-one (tioxolone), 2,7-dimethylthianthrene (mesulfen), menthol, and pramoxine hydrochloride, and combinations thereof.

In one embodiment, the compositions of the present invention include an anti-viral agent. Examples of anti-viral agents include, but are not limited to, imiquimod, podofilox, podophyllin, interferon alpha, acyclovir, famcyclovir, valcyclovir, reticulos and cidofovir.

In one embodiment, the compositions of the present invention includes antiseborrea or sebum inhibition agents such as elubiol.

In another embodiment, the composition includes surfactants such as but not limited to sodium methyl cocoyl taurate, sodium lauryl sulfonate, sodium lauryl sulfoacetate, sodium cocoyl isethionate, sodium olefin sulfonate, dioctyl sodium sulfosuccinate, amphoteric materials such as cocamodopropyl hydroxyl sultaine, structure surfactants and combinations thereof.

In one embodiment, the compositions of present invention include an anti-dandruff agent. Examples of anti-dandruff agents include but are not limited to zinc pyrithione, elubiol, coal tar, salicylic acid or selenium sulfide, sulphur, ketoconazole, corticosteroids such as fluocinolone acetonide, caffeine and combinations thereof.

In one embodiment, the composition of the present invention includes active agents for treating keratosis pilaris. Examples of active agents for treating keratosis pilaris include but are not limited to fluoracil, Imiquimod, aminolevulinic acid and combinations thereof.

In one embodiment, the compositions of the present invention include an anti-inflammatory agent. Examples of anti-inflammatory agents, include, but are not limited to, non-steroidal and steroidal anti-inflammatory agents such as corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, tluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, and triamcinolone, and combinations thereof. Examples of non-steroidal anti-inflammatory agents include but not limited to COX inhibitors, LOX inhibitors, and p38 kinase inhibitors, immunosuppresant agents such as cyclosporin, and cytokine synthesis inhibitors. Other natural antiinflammatories include, but are not limited to, extracts of feverfew, boswellia, aloe vera, chamomille, lavender, soy, or oats, beta-glucan, and totarol. Other active agents include, but are not limited to, wound healing enhancing agents such as calcium alginate, collagen, recombinant human platelet-derived growth factor (PDGF) and other growth factors, ketanserin, iloprost, prostaglandin E.sub.1 and hyaluronic acid; scar reducing agents such as mannose-6-phosphate; analgesic agents; debriding agents such as papain, and enzymatic debriding agents; and anesthetics such as lidocaine and benzocaine. In one embodiment, the composition comprises one or more of menthol, camphor, an antihistamine, or a local anesthetic such as tetracaine, lidocaine, prilocaine, benzocaine, bupivacaine, mepivacaine, dibucaine, etidocaine, butacaine, cyclomethycaine, hexylcaine, proparacaine, and lopivacaine, capsaicin, or oatmeal.

In one embodiment, the amount of anti-inflammatory agent, anti-viral agent, antipsoroiatic agent and/or other active agent in the composition is from about 0.001% to about 10%, such as from about 0.01% to about 5% such as from about 0.05% to about 2% by weight, based on the total weight of the composition.

In one embodiment, the composition of the present invention includes hair growth regulating agents for helping or retarding hair growth, to treat alopecia and hirsutism, such as a 5-alpha reductase inhibitor. Examples of 5-alpha reductase inhibitors include but are not limited to finasteride, dutasteride, ketoconazole, minoxidil and natural extracts such as but not limited to soy and isoflavones.

The compositions of the present invention may further include an alcohol. Examples of suitable alcohols include, but are not limited to, ethyl alcohol. In one embodiment, the composition includes less than 40%, such as from about 0.01% to about 40%, for example from about 0.1% to about 30%, or from about 1% to about 20% by weight, of alcohol based on the total weight of the composition.

In one embodiment, the composition includes a nonionic surfactant. Examples of nonionic surfactants are disclosed on pages 2955-2976 of the International Cosmetic Ingredient Dictionary and Handbook, eds. Wenninger and McEwen, (The Cosmetic, Toiletry, and Fragrance Assoc., Washington, D.C., 9.sup.th Edition, 2002) (hereinafter "CTFA Handbook").

In one embodiment, the composition includes a skin lightening agent. Examples of skin lightening agents include, but are not limited to, retinoids such as retinol, adapalene, retinal, tretinoin (retinoic acid, Retin-A), isotretinoin, alitretinoin, etretinate and its metabolite acitretin, tazarotene, bexarotene and adapalene and their derivatives and combinations, extracts of soy, licorice, lilly and tyrosine inhibitors such as hydroquinone, ascorbyl glucoside, kojic acid, calcium D-pantetheine-S-sulfonate, arbutin, magnesium ascorbyl phosphate, pantothiol, dihydrolipoic acid, arlatone glycolic and fruit acids (a-hydroxy acids or AHAs), lactic acid, citric acid, mandelic acid, b-hydroxy acid such as salicylic acid, beta-hydroxybutyric acid, carnitine, 3-hydroxypropionic acid, and their derivatives and their derivatives and combinations thereof.

In one embodiment, the composition may include light effecting ingredients, color modifiers such as iron oxide, titanium oxide, zinc oxide, silica, clay such as kaolin, starch such as corn starch and rice starch, color rendering or photochromatic materials such as but not limited to pearlescent materials, diffractive pigments obtained with high and low index dielectric materials such as TiOx, TiOx and FeOx.

In another embodiment, the composition of the present invention will include film forming or porous polymers that create a micro-thin layer to further reduce irritations, enhance stability and sustained release of actives such as BPO.

In another embodiment, the composition may include compounds that improve aesthetics of the composition and the release rate of active ingredients. What is meant by "improve aesthetics" may include but is not limited to improved feel, texture, smoothness of the composition. Examples of suitable compounds include but are not limited to silica, hydrated silica, colloidal silica, aluminum sulfate, and combinations thereof.

In another embodiment, the composition may include comprise cooling or warming agents. Examples of cooling or warming agents include but are not limited to menthol, zeolites and combinations thereof.

In one embodiment, the composition of the present invention has a pH greater that about 3.0 and a pH less than about 10.0 such as less than about 8.0, such as less than about 7.0.

The compositions useful in the present invention involve formulations suitable for topical application to skin. The composition may further include a cosmetically-acceptable topical carrier. The cosmetically-acceptable topical carrier may comprise from about 50% to about 99%, by weight, of the composition (e.g., from about 80% to about 95%, by weight, of the composition).

Optionally, minor ingredients such as fragrances, colorants, can generally be present in minor amounts, such as less than 10% by weight and preferably less than 2% wt/wt. Such additives should be soluble or miscible with either non-aqueous phase or aqueous phase/water.

The compositions may be made into a wide variety of product types that include but are not limited to solid and liquid compositions such as lotions, creams, gels, sticks, sprays, shaving creams, ointments, cleansing liquid washes and solid bars, pastes, powders, mousses, masks, peels, make-ups, and wipes. These product types may comprise several types of cosmetically acceptable topical carriers including, but not limited to solutions, emulsions (e.g., microemulsions and nanoemulsions), gels, solids and liposomes). The compositions may be used in conjunction with other devices such as skin abrading, skin messaging, electrostimulation devices, light-therapy devices, ultrasound devices, radio frequency devices, thermal/cooling devices, and micro-penetration devices. The following are non-limitative examples of such carriers. Other carriers can be formulated by those of ordinary skill in the art.

The compositions useful in the present invention can be formulated as solutions. Solutions typically include an aqueous solvent (e.g., from about 50% to about 99% or from about 90% to about 95% of a cosmetically acceptable aqueous solvent).

Compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. See CTFA Handbook which contains numerous examples of suitable materials.

Topical compositions useful in the subject invention may be formulated as but not limited to a lotion, comprising an humatants such as propylene glycol, butylenes glycol, hexylene glycol, glycerin, or those listed in CTFA handbook.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in the CTFA Handbook pp. 1693-1697.

The compositions useful in the present invention may be formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, CTFA Handbook, pp. 1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprise between about 0.1% and 5%, by weight, of such gelling agents.

In one embodiment, the composition is anhydrous. In one embodiment, such anhydrous composition is exothermic upon application.

The compositions of the present invention can also be formulated into a solid formulation (e.g., a wax-based stick, soap bar composition, powder, a wipe containing powder, or a dressing).

The compositions useful in the subject invention may contain, in addition to the aforementioned components, a wide variety of additional oil-soluble materials and/or water-soluble materials conventionally used in compositions for use on the skin at their art-established levels.

The compositions may be applied as needed and/or as part of a regular regimen ranging from application once a week up to one or more times a day (e.g., twice a day). The amount used will vary with the age and physical condition of the end user, the duration of the treatment, the specific compound, product, or composition employed, the particular cosmetically-acceptable carrier utilized, and like factors.

Several non-limiting examples are described below.

### Example 1:

A composition according to the invention comprising the ingredients in Table 1 was prepared by first adding benzoyl peroxide and approximately 100 g of water along with the versene NA to a beaker with mixing. The pH of the benzoyl peroxide phase was adjusted to 5.2-5.5 with potassium hydroxide, and then the mixture was placed over low heat until it reached a temperature of 30-35 °C. In another beaker, the Ceraphyl 28 was gently heated to 30-35 °C, then the DC246 fluid was hand stirred in and this premix was stirred into the benzoyl peroxide phase, which was removed from the heat and allowed to mix for no less than 30 minutes. The brij 30, glycerin, phospholipid PTC and ceraphyl 41 were then added, along with the remaining water. Next the carbopol and hydroxypropyl methylcellulose were added and stirred until well combined. The BZK was then added, and the pH of the system was adjusted to 5.2-5.5.

**Table 1**

| | |
|---|---|
| Purified Water | 74.11 % |
| Benzoyl Peroxide | 10.00 % |
| Versene NA | 0.40 % |
| Brij 30 | 3.00 % |
| Dow Corning 246 Fluid | 3.00 % |
| Glycerin | 5.00 % |
| Arlasilk Phospholipid PTC | 0.75 % |
| Ceraphyl 41 | 1.50 % |
| Ceraphyl 28 | 1.00 % |
| Carbopol | 1.00 % |
| Hydroxypropyl Methylcellulose | 0.40 % |
| Benzalkonium Chloride | 0.09 % |
| Potassium Hydroxide (20% Solution) | 0.40 % |

### Example 2:

The composition of Example 1 was tested in a clinical study for level of BPO deposition as follows. Five healthy male subjects of age from 30 to 50 were recruited. A trained operator, using latex exam gloves, applied a known quantity (approximately 2 milligram /cm² measured with a pipette) of Clearasil® Maximum Strength Acne Treatment Cream (10% BPO), commercially available from Reckitt Benckiser (comparative) and the composition of Example 1 onto pre-selected test areas of each panelist. The operator then rubbed the test areas to distribute the product as uniformly as possible. The samples were allowed to stay on the test areas for about 1 hour. Ten D-squame tapes were used to remove the surface residues of the two compositions. A dye solution was then applied to the tape stripped test sites. A microscopic image was taken and used to count the number of stained pores on each test site.

The results are shown in Table 2, which indicates the number of follicles with BPO deposition in a 1" area diameter circle on a subject's skin. The results of this clinical study show benzoyl peroxide deposition in a larger number of follicles when the composition of Example 1 was applied topically, compared with the Clearasil® product.

**Table 2**

| Subject # | Clearasil® | Example 1 Composition |
|---|---|---|
| 1 | 24 | 15 |
| 2 | 53 | 78 |
| 3 | 23 | 71 |
| 4 | 50 | 87 |
| 5 | 26 | 39 |
| | | |
| Average | 35.2 | 58.0 |
| Std Dev | 15.0 | 30.1 |

### Example 3:

The composition of Example 1 was tested for acne efficacy in a clinical study as follows. Participants were asked to use the composition of Example 1 and Clean & Clear Persa-Gel-10, Maximum Strength (10% benzoyl peroxide) commercially available from Johnson & Johnson Consumer Companies, Inc. in a split face manner, twice daily for seven consecutive days. Photographic images of the subjects were taken at baseline and day 7. The images of were submitted to a trained dermatologist for clinical grading of the parameters "Appearance of Lesions" and "Skin Redness." Each split face image from day 7 was graded against the same treatment side of face at baseline on a sliding scale or 0 to 4, with O indicating least severe and 4 indicating most severe.

The results are shown in Table 3 below, which illustrates directional improvement in the appearance of lesions and skin redness using the composition of Example 1 versus Clean & Clear Persa-Gel 10, Maximum Strength.

**Table 3**

| | Appearance of Lesions | | Skin Redness | |
|---|---|---|---|---|
| | Example 1 | Persagel | Example 1 | Persagel |
| | -2 | 0 | -2 | 0 |
| | 0 | 2 | 0 | 1 |
| | 0 | 2 | 0 | 3 |
| | -3 | -1 | -1 | -1 |
| | 0 | -1 | 0 | -1 |
| | -1 | 1 | 0 | 1 |
| | 1 | 1 | -1 | 1 |
| Average | -0.71 | 0.57 | -0.57 | 0.57 |
| p value t-test | | | | |
| | | 0.095 | | 0.083 |

### Example 4:

The composition of Example 1 and Clearasil® Maximum Strength Acne Treatment Cream (10% BPO) (Reckitt Benckiser) were tested for irritation as follows. Normal human-derived epidermal keratinocytes (NHEK) skin equivalent samples were used. Six micro liters of each composition were topically applied to the skin equivalents and then spread across the surface until visually even distribution was attained. The treated skin equivalents were incubated for 24 hours and then tested for irritation markers. The IL-1α response in skin equivalent samples for the composition of Example 1, the Clearasil® product, and an untreated control are shown in Figure 1. The composition of Example 1 showed a lower irritation response of less than 250 IL-1α compared to the Clearasil® product, which had a response more than 350 IL-1α after 24 hours of contact time.

### Example 5

A 5% BPO composition was prepared in using the same ingredients as Example 1, but containing only 5% BPO. The composition was tested clinically versus BenzaClin®, a 5% commercially available acne treatment product (Aventis, NJ, USA). In this clinical study, 20 healthy subjects of mild to moderate acne used the 5% BPO composition of the invention and BenzaClin® in a split face test for 10 consecutive weeks. The products were used twice daily. At the end of the study, the composition of the invention showed less dryness and peeling. Both the composition of the invention and BenzaClin® showed significant reduction versus their baseline in total lesion count and total inflammatory lesion count (papules, pustules and cysts). There were no statistical differences between the products for any acne treatment efficacy parameters.

### Example 6

A composition of the present invention (3.0% BPO) is prepared in a similar manner as Example 1 using the ingredients shown in Table 5. About 0.6 g of the formula from Table 5 is added to a 2x2 square inch nonwoven pad (Poloxamer 182) and the pad is sealed in an aluminum pouch or a glass jar.

**Table 5**

| | |
|---|---|
| Benzoyl Peroxide | 3.00 % |
| Versene NA | 0.20 % |
| Brij 30 | 0.500 % |
| Dow Corning 246 Fluid | 0.500 % |
| Hydrated silica | 1.00 % |
| Cetyl lactate | 1.00 % |
| Carbomer | 0.2 % |
| Glycerin | 1.00 % |
| Disodium lauryl sulfosuccinate | 10% |
| Potassium Hydroxide (20% Solution) | QS |

### Example 7

A composition of the present invention (2.5 % BPO) was prepared in a similar manner as Example 1 using the ingredients shown in Table 6.

**Table 6**

| | |
|---|---|
| Purified Water | 80.96 % |
| Benzoyl Peroxide | 2.50 % |
| EDTA | 0.40 % |
| Laureth-30 | 3.00 % |
| C13-16 Isoparaffin,C12-14 Isoparaffin,, | 3.00 % |
| C13-15 Alkane) | |
| Glycerin | 5.00 % |
| Arlasilk Phospholipid PTC | 0.75 % |
| Cetyl lactate | 1.50 % |
| C12-15 Alkyl lactate | 1.00 % |
| Carbomer | 1.00 % |
| Hydroxypropyl Methylcellulose | 0.40 % |
| Benzalkonium Chloride | 0.09 % |
| Potassium Hydroxide (20% Solution) | 0.40 % |

### Example 8

A benzoyl peroxide assay was performed by high performance liquid chromatography (HPLC) on the composition of Example 1. HPLC indicated a recovery of more than 99% of the Example 1 composition after storing at room temperature (22°C) for more than 100 days, indicating good BPO stability.

While this specification describes the principles of the present invention, it is to be understood that other aspects, advantages and modifications within the scope of the invention will be apparent to those skilled in the art.

## Claims

1. A composition for treating acne comprising an anti-acne agent, a mixture of a co-solvent and an ester, and a topical carrier, wherein said ester has a melting point of at least about 30° C and said mixture is substantially immiscible with said topical carrier.

2. A composition according to claim 1, wherein said anti-acne agent is selected from the group consisting of salicylic acid, benzoyl peroxide, sulphur, retinoic acid, azelaic acid, clindamycin, adapalene, erythromycin, sodium sulfacetamide, aluminium chloride, resorcinol, dapsone, aluminum oxide, and combinations thereof.

3. The composition according to claim 1 or claim 2, wherein said anti-acne agent is benzoyl peroxide.

4. The composition according to claim 3, wherein said benzoyl peroxide is present in the amount of from about 0.1% to about 15% by weight of the composition.

5. The composition according to any preceding claim, wherein said co-solvent has a flashing point lower than 150° C.

6. The composition according to any preceding claim, wherein said co-solvent is selected from the group consisting of volatile cyclic polysiloxanes, volatile linear polysiloxanes, branched polysiloxanes containing methyl groups, aliphatic hydrocarbons and combinations thereof.

7. The composition according to any preceding claim, wherein said co-solvent is present in the amount of from about 0.1% to about 30% by weight of the composition.

8. The composition according to any preceding claim, wherein said ester is selected from the group consisting of C16 or longer linear carbon chain alkyl lactates, hydrocarbon waxes, rice bran wax, beeswax, copernicia cerifera wax, sunflowseedcetyl lactate, myristate lactate, wax, euphobia wax, shea butter, cetyl stearate, cetyl alcohol, ethylhexylglycerin, petrolatum, cholesterol, their derivatives, and combinations thereof.

9. The composition according to any preceding claim, wherein said ester is present in the amount of from about 0.1% to about 15% by weight of the composition.

10. The composition according to any preceding claim, wherein said composition further comprises a phospholipid.

11. The composition according to claim 10, wherein said phospholipid is selected from the group consisting of sodium coco PG-Dimonium Chloride phosphate, cocamidopropyl PG-Dimonium Chloride Phosphate, myristamidopropyl PG-Dimonium Chloride phosphate and combinations thereof.

12. The composition according to any preceding claim, wherein said composition comprises a lactate.

13. The composition according to claim 12, wherein said lactate is selected from the group consisting of C16-C35 alkyl lactates and combinations thereof.

14. A composition according to any one of claims 1 to 13 for use in treating a follicular disease said use comprising topically applying the composition to an area of skin in need of such treatment.
